# EUROPEAN PATENT APPLICATION

(11) **EP 4 427 807 A1**
(43) Date of publication of application: **11.09.2024**
(21) Application number: 23382211.3
(22) Date of filing: 07.03.2023
(51) Int. Cl.: A61P 25/02, A61P 25/08, C07D 255/04, A61K 31/395

(54) **N-BRIDGED DIAZOCINE ANALOGUES OF CARBAMAZEPINE TO CONTROL NEURONAL ACTIVITY WITH LIGHT AND APPLICATIONS THEREOF**

(71) Applicant: Fundació Institut de Bioenginyeria de Catalunya (IBEC), 08028 Barcelona (ES); Institució Catalana de Recerca i Estudis Avançats, 08010 Barcelona (ES); Consorcio Centro de Investigación Biomédica en Red (CIBER), 28029 Madrid (ES)
(72) Inventor: GOROSTIZA LANGA, Pau, E-08028 Barcelona (ES); MATERA, Carlo, I-20133 Milan (IT); CAMERIN, Luisa, E-08028 Barcelona (ES); RIEFOLO, Fabio, E-08025 Barcelona (ES); MALIEIEVA, Galyna, E-08028 Barcelona (ES); GOMILA JUANEDA, Alexandre, E-08028 Barcelona (ES)
(74) Representative: ABG Intellectual Property Law, S.L.

(57) **Abstract**

The present invention relates to a compound of formula (I) or a pharmaceutically acceptable salt or stereoisomer thereof, to pharmaceutical compositions comprising said compound, and to their use in the treatment and/or prevention of epilepsy or neuropathic pain.

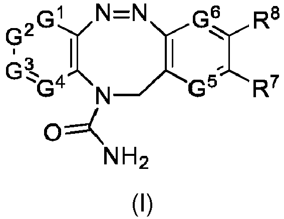

## Description

### FIELD OF THE INVENTION

The present invention relates to compounds useful for the treatment neurological conditions that involves excessive neuronal activity or signaling, such as epilepsy and neuropathic pain, to the use of said compounds and pharmaceutical compositions as a medicament, in particular for the treatment and/or prevention of epilepsy and neuropathic pain.

### BACKGROUND OF THE INVENTION

Epilepsy is a common neurological disorder in which abnormal electrical discharges (repetitive action potentials) in the central nervous system (CNS) cause recurrent unprovoked seizures. Abnormal generation of action potentials in damaged nerves is also involved in pain.

It is widely established that antiepileptic drugs, such as carbamazepine, exert their effect through decreasing the frequency of action potentials generation [Elliott P. Action of antiepileptic and anaesthetic drugs on Na- and Ca-spikes in mammalian non-myelinated axons. Eur J Pharmacol. 1990 Jan 10;175(2):155-63].

Carbamazepine was first reported to be effective in treating trigeminal neuralgia in 1962 [Blom, S. (1962); Trigeminal neuralgia: its treatment with a new anticonvulsant drug (G-32883). Lancet, 1(7234):839-840]. Carbamazepine presents the ability to block generation of action potentials [McLean, M. J., & Macdonald, R. L. (1986). Carbamazepine and 10,11-epoxycarbamazepine produce use-and voltage-dependent limitation of rapidly firing action potentials of mouse central neurons in cell culture. Journal of Pharmacology and Experimental Therapeutics, 238(2), 727-738]. Carbamazepine is a broadly used antiepileptic drug, it is currently first-line treatment for focal onset seizures in the USA and Europe [Nevitt SJ, Marson AG, Tudur Smith C. Carbamazepine versus phenytoin monotherapy for epilepsy: an individual participant data review. Cochrane Database Syst Rev. 2019 Jul 18;7(7):CD001911].

Antiepileptic drugs have also been reported to counteract the neuropathic pain through inhibiting generation of high-frequency action potentials by damaged nerve [Rogawski, M., Löscher, W. The neurobiology of antiepileptic drugs for the treatment of nonepileptic conditions. Nat Med 10, 685-692 (2004)]. In particular, carbamazepine also proved efficacious in a treatment of neuropathic pain in a number of randomized placebo-controlled clinical trials [Campbell FG, Graham JG, Zilkha KJ. Clinical trial of carbazepine (tegretol) in trigeminal neuralgia. J Neurol Neurosurg Psychiatry. 1966 Jun;29(3):265-7; and McQuay H, Carroll D, Jadad AR, Wiffen P, Moore A. Anticonvulsant drugs for management of pain: a systematic review. BMJ. 1995 Oct 21;311(7012):1047-52].

However, some serious side effects of systemic administration of carbamazepine have been reported [Wiffen PJ, Derry S, Moore RA, Kalso EA. Carbamazepine for chronic neuropathic pain and fibromyalgia in adults. Cochrane Database Syst Rev. 2014 Apr 10;2014(4):CD005451], such as nausea, vomiting, dizziness, somnolence, diplopia, vertigo, and ataxia. Even highly specific drugs that bind only to a particular type of receptors, can produce side effects if the receptor is expressed at multiple locations, as it is impossible to control the drug distribution within the body and prevent its interaction with the receptors that are localized outside of the targeted organ.

Thus, there remains a need for new treatment options for neurological conditions that involve excessive neuronal activity or signaling, such as epilepsy and pain signaling in the spinal cord and brain (in particular neuropathic pain), that overcome the drawbacks of the prior art.

### SUMMARY OF THE INVENTION

The inventors have surprisingly found new compounds that may be administered systemically and produce neither therapeutic nor adverse effects in the absence of illumination to the nervous system, whereas upon illumination they are capable of inhibiting or decreasing the frequency of action potentials generation and are therefore suitable for the treatment of neurological conditions that involve excessive neuronal activity or signaling, such as epilepsy and pain signaling in the spinal cord and brain (in particular neuropathic pain). These compounds are light activated and, thus, the neuroinhibitory effects are only localized at the region of illumination and on demand, decreasing the incidence of the severe adverse effects. As shown in the examples, in zebrafish larvae, the compounds of the invention are not toxic, do not alter zebrafish larvae behaviour in the dark, and produce behavioural responses upon illumination.

Thus, in the first aspect the present invention relates to a compound of formula (I): 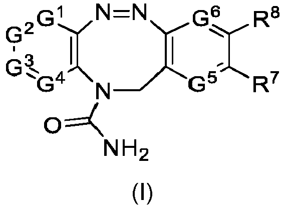 wherein:
G¹ is selected from nitrogen atom and CR¹,
G² is selected from nitrogen atom and CR²,
G³ is selected from nitrogen atom and CR³,
G⁴ is selected from nitrogen atom and CR⁴,
G⁵ is selected from nitrogen atom and CR⁵,
G⁶ is selected from nitrogen atom and CR⁶,
R¹, R², R³, R⁴, R⁵, R⁶, R⁷ and R⁸ are independently selected from hydrogen and halogen atom,
with the proviso that no more than one of G¹, G², G³ and G⁴ is a nitrogen atom, and
with the proviso that no more than one of G⁵ and G⁶ is a nitrogen atom,
or a pharmaceutically acceptable salt or stereoisomer thereof.

In a second aspect, the present invention relates to a pharmaceutical composition comprising a compound according to the first aspect, and a pharmaceutically acceptable excipient.

In a third aspect, the present invention relates to a compound according to the first aspect or a pharmaceutical composition according to the second aspect, for use in medicine.

In a fourth aspect, the present invention relates to a compound according to the first aspect or a pharmaceutical composition according to the second aspect, for use in the treatment and/or prevention of a disease selected from the group consisting of epilepsy and neuropathic pain.

### DESCRIPTION OF THE FIGURES

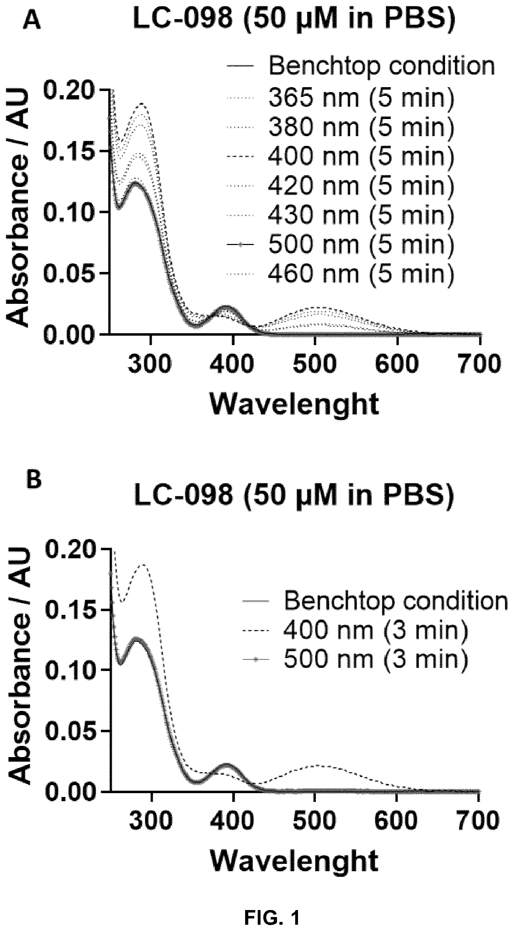

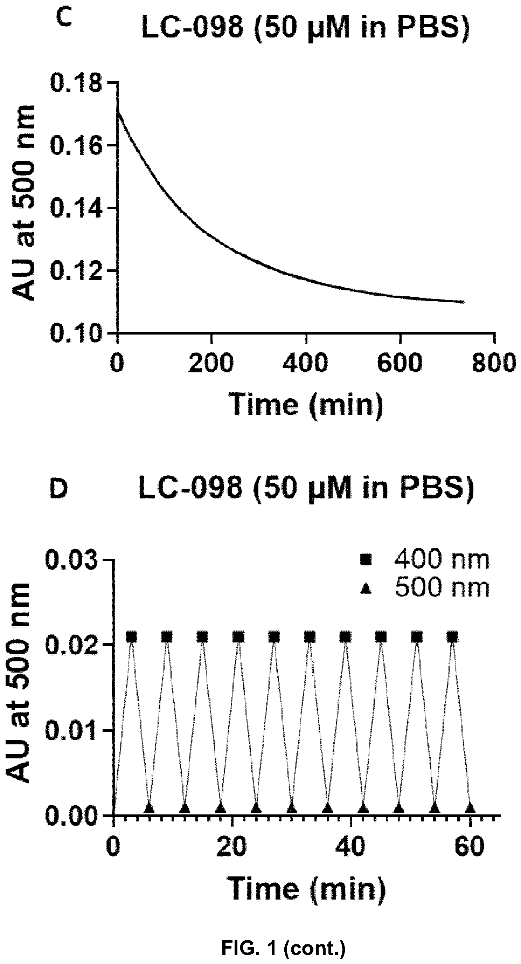

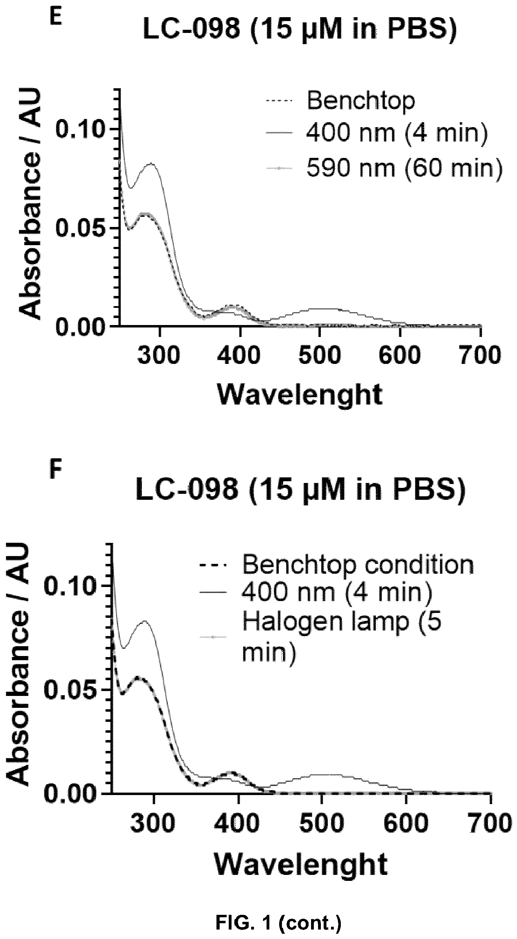

Figure 1 shows the photochemical behaviour in PBS solution 0.01M. A) Absorption spectra at benchtop conditions and under different condition of illumination. B) Absorption spectra obtained at maximum conversion with the most efficient wavelengths. C) Kinetic profile for the thermal relaxation time from Z to E in dark conditions at 25°C. D) Resistance to several cycles of illumination showing no loss in absorbance. E) Absorption spectra obtained upon irradiation with LEDs emitting at 400 nm and 590 nm. F) Absorption spectra obtained upon irradiation with LED emitting at 400 nm and with halogen lamp.

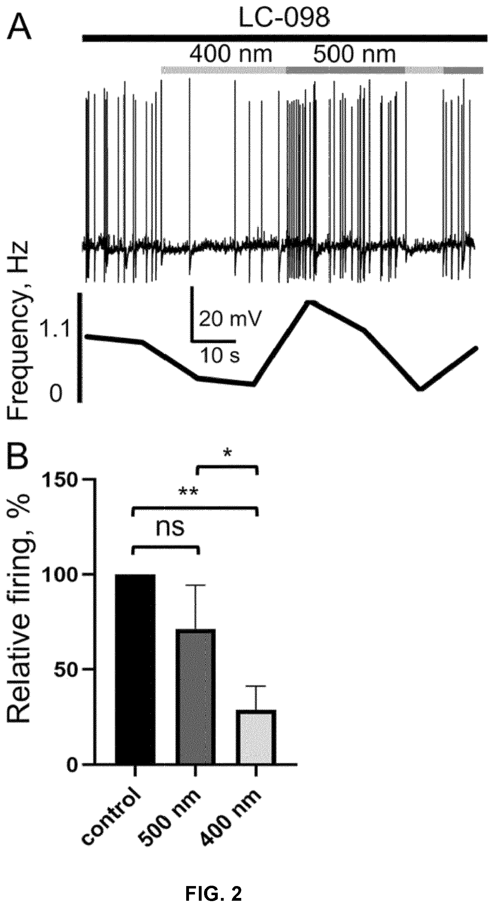

Figure 2 shows the photocontrol of action potential firing by the compound of formula (II). A. Current clamp recording of compound of formula (II) mediated photocontrol of action potentials in cultured hippocampal neurons. Application of the compound of formula (II) is indicated by black bar above the trace, periods of illumination with 400 nm and 500 nm lights are indicated by light gray and dark gray bars respectively. B. Cumulative graph demonstrating the effect of 500 nm (dark gray column) and 400 nm (light gray column) illumination in the presence of the compound of formula (II) at the neuronal firing comparing to the control. * - P < 0.05; ** - P < 0.01; ns - not significant.

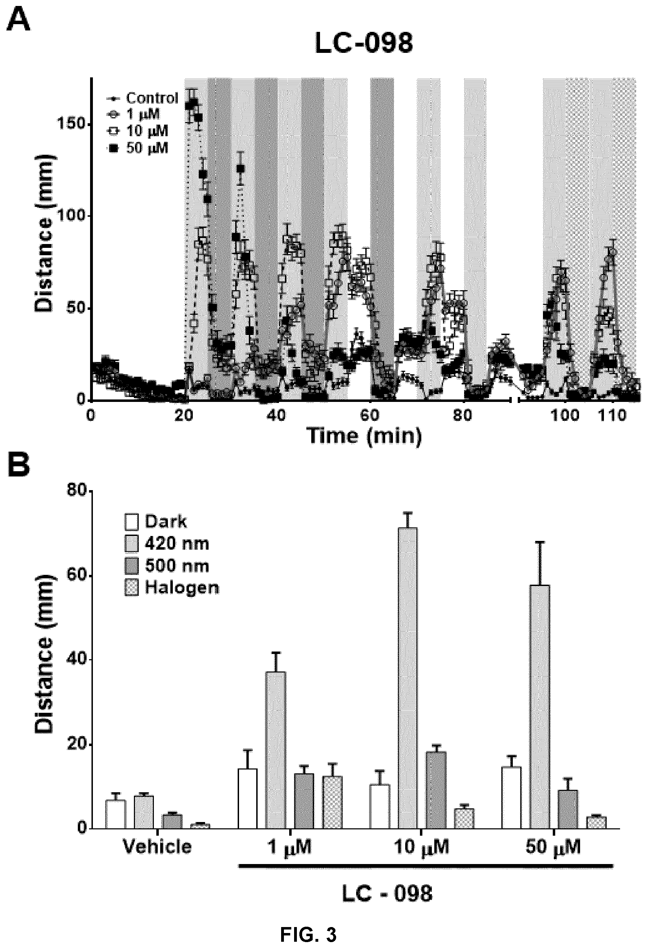

Figure 3 shows the behavioural characterisation of the compound of formula (II) in 7 days post-fertilisation Tupfel-Long zebrafish larvae. A. Swimming activity (mm per minute) trajectories for vehicle (1 % DMSO, black trace, full circles) and treated with the compound of formula (II) (1 µM, void circles, grey line; 10 µM, void squares, dashed line; 50 µM, full squares, dotted line) zebrafish. Larvae were exposed to an initial 20 minutes period in full darkness (clear areas) (relaxing period and benchtop E state for treated groups) followed by alternating 5 minutes illumination periods in order to potentiate the photoswitchable compound between its E (500 nm or halogen warm white light, grey and patterned areas) and Z (420 nm, light grey) states. Error bars depict S.E.M. per each group (n = 24). B. Quantification of the average distance swum by each group over all light cycles of 420 nm (25 minutes integration), 500 nm (25 minutes) and halogen lamp (10 minutes).

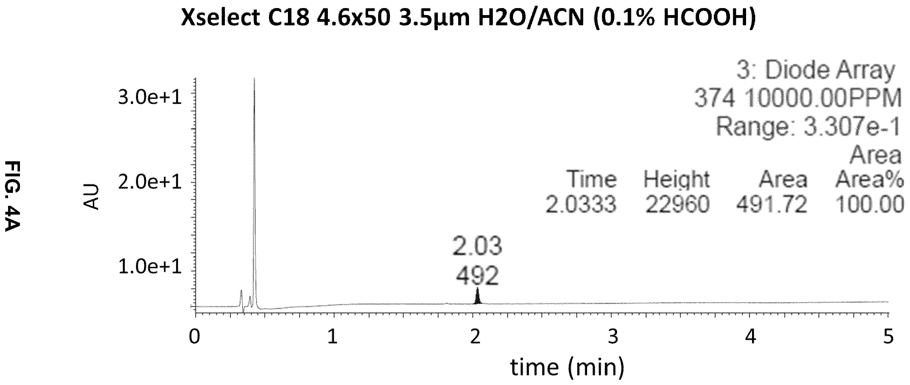

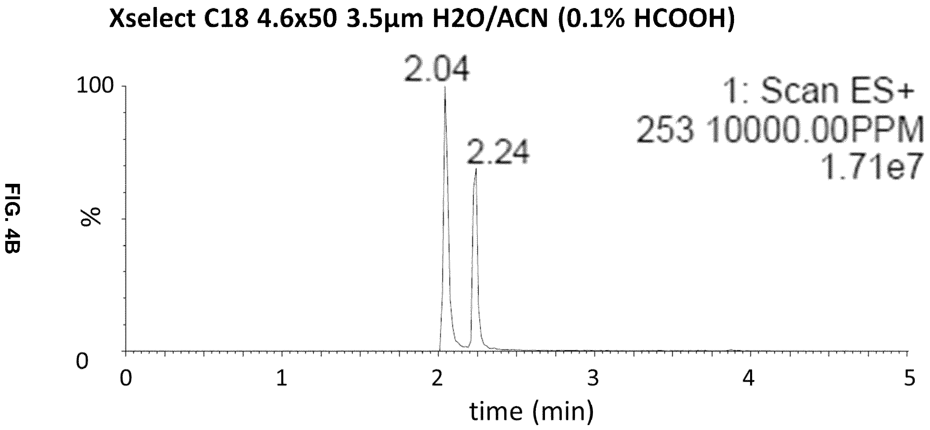

Figure 4 shows the quantification of photostationary state for compound LC-098 (compound of formula (II)) by HPLC analysis. At benchtop dark condition, the integrations are showing the ratio between the two isomers: ≥ 99% *cis, trans* isomer not detected. FIGURE 4A shows the Diode array spectra at the isosbestic wavelength (374 nm) with the integrations. FIGURE 4B shows the mass spectra detecting m/z 253.

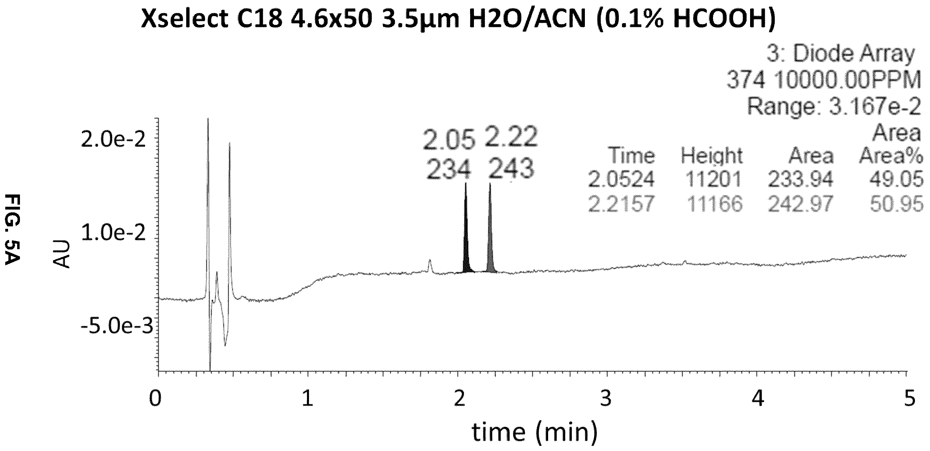

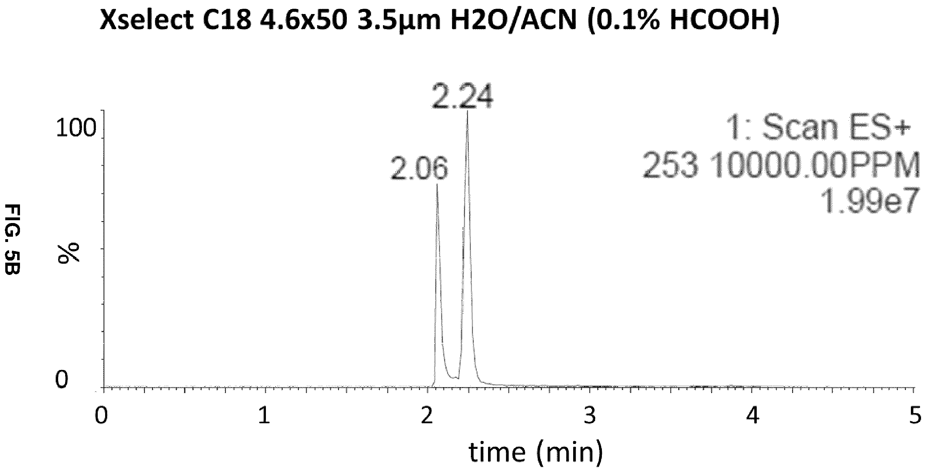

Figure 5 shows the quantification of photostationary state for compound LC-098 (compound of formula (II)) by HPLC analysis. After irradiation at 400 nm for 3 minutes, the integrations are showing the ratio between the two isomers: 49% *cis,* 51% *trans.* FIGURE 5A shows the diode array spectra at the isosbestic wavelength (374 nm) with the integrations. FIGURE 5B shows the mass spectra detecting m/z 253.

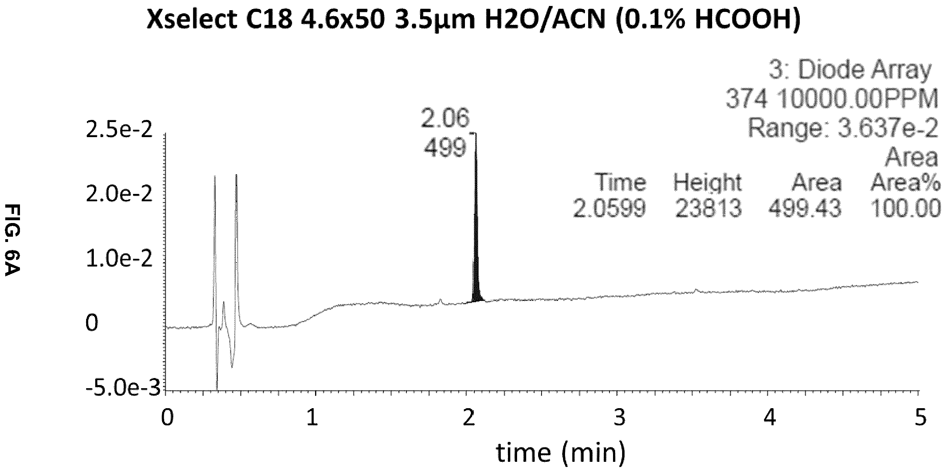

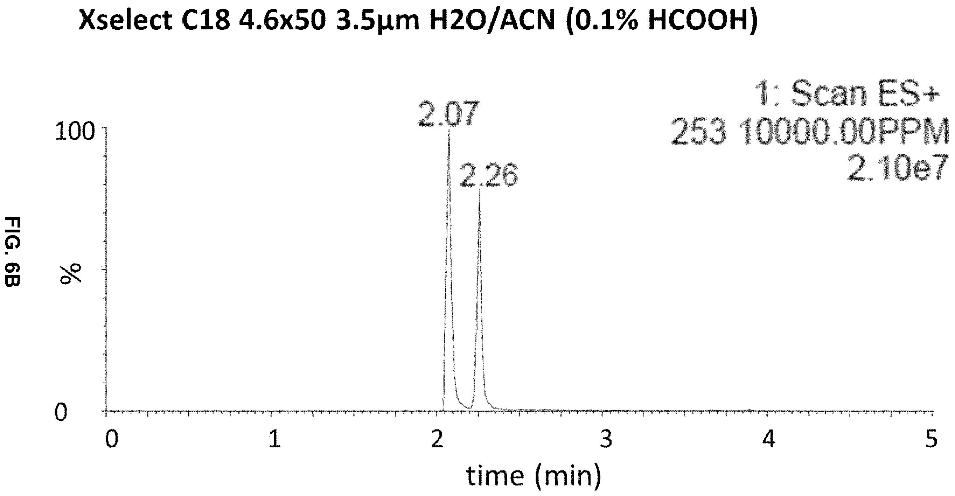

Figure 6 shows the quantification of photostationary state for compound LC-098 (compound of formula (II)) by HPLC analysis. After irradiation at 500 nm for 3 minutes, the integrations are showing the ratio between the two isomers: ≥ 99% *cis, trans* isomer not detected. FIGURE 6A shows the diode array spectra at the isosbestic wavelength (374 nm) with the integrations. FIGURE 6B shows the mass spectra detecting m/z 253.

### DETAILED DESCRIPTION OF THE INVENTION

### Compounds of formula (I)

In the first aspect, the present invention relates to a compound of formula (I): 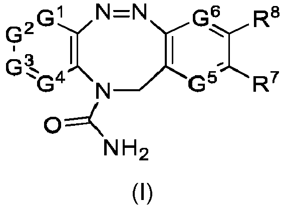 wherein:
G¹ is selected from nitrogen atom and CR¹,
G² is selected from nitrogen atom and CR²,
G³ is selected from nitrogen atom and CR³,
G⁴ is selected from nitrogen atom and CR⁴,
G⁵ is selected from nitrogen atom and CR⁵,
G⁶ is selected from nitrogen atom and CR⁶,
R¹, R², R³, R⁴, R⁵, R⁶, R⁷ and R⁸ are independently selected from hydrogen and halogen atom,
with the proviso that no more than one of G¹, G², G³ and G⁴ is a nitrogen atom, and
with the proviso that no more than one of G⁵ and G⁶ is a nitrogen atom,
or a pharmaceutically acceptable salt or stereoisomer thereof.

The term "halogen" as used herein alone or as part of another group refers to chlorine, bromine, fluorine, and iodine, preferably fluorine.

As used herein, the term "pharmaceutically acceptable salt" embraces salts with a pharmaceutically acceptable acid or base, which are synthesized from the parent compound which contains an acidic moiety by addition of a pharmaceutically acceptable base, or which are synthesized from the parent compound which contains a basic moiety by addition of a pharmaceutically acceptable acid. Pharmaceutically acceptable acids include both inorganic acids, for example, hydrochloric (HCl), sulfuric (H₂SO₄), phosphoric (H₃PO₄), diphosphoric (H₄P₂O₇), hydrobromic (HBr), hydroiodic (HI), and nitric acid (HNO₃), and organic acids, for example, citric, fumaric, maleic, malic, mandelic, ascorbic, oxalic, succinic, tartaric, benzoic, acetic (AcOH), methanesulfonic, ethanesulfonic, benzenesulfonic, or p-toluenesulfonic acid. Pharmaceutically acceptable bases include alkali metal (e.g., sodium or potassium) and alkali earth metal (e.g., calcium or magnesium) hydroxides and organic bases, such as alkyl amines, arylalkyl amines, and heterocyclic amines.

All stereoisomers of the compounds of this invention are contemplated either alone or as mixtures thereof. Stereoisomers refer to compounds having stereogenic centres, e.g. enantiomers, diastereomers, meso and racemic forms, and to compounds having different substituents on the atoms linked to form a multiple bond, such as a double or triple bond (e.g. -HC=CH-, -C≡C-, -N=N-, etc.), i.e. *cis* (Z) and *trans* (E) isomers. In particular, the compounds of the invention have E-Z stereoisomerism around the -N=N-double bond. When diastereomeric or enantiomeric products are prepared, they can be separated by conventional methods, for example, chromatographic or functional crystallization. When *Z* and *E* isomers of the compounds of formula (I) according to the present invention are prepared they can be reversibly interconverted from one isomer to the other isomer by irradiation at the appropriate wavelength, i.e., to obtain the *E* isomer from the *Z* isomer, a wavelength between 380 nm and less than 430 nm may be used, preferably from 380 nm to 420 nm, more preferably from 400 nm to 420 nm, and to obtain the *Z* isomer from the *E* isomer irradiation at a wavelength of at least 500 nm, preferably between 500 nm and 600 nm may be used. The *Z* isomer of the compounds of formula (I) is the inactive form, whereas the *E*-isomer of the compounds of formula (I) is the active form. In one embodiment, the compounds of formula (I) are in the form of the *E*-isomer. In another embodiment, the compounds of formula (I) are in the form of the *Z*-isomer. The *Z*-isomer is then irradiated at the appropriate wavelength (as described above) to provide the active *E*-isomer.

An example of the *Z-E* isomerization around the N=N bond induced by illumination of a specific compound of formula (I) (namely, the compound of formula (II)) is shown below:

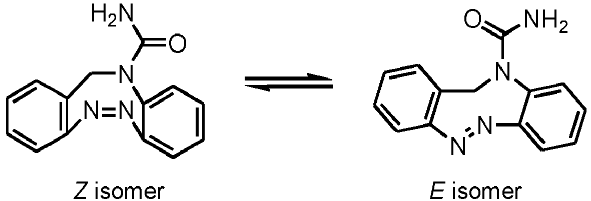

Moreover, the *Z* isomer can adopt two boat conformations wherein the amide substituent in the central ring is in axial or in equatorial position, i.e. boat axial and boat equatorial, as shown below. In the context of the present invention, when referring to the *Z* isomer, both conformations (boat axial and boat equatorial) are included. Also, the *E* isomer can adopt different conformations, two twist conformations and two chair conformations, as shown below. In the context of the present invention, when referring to the *E* isomer, all twist and chair conformations are included. An example of the boat (axial and equatorial), twist and chair conformations adopted by the *Z* and *E* isomers of a specific compound of formula (I) (namely, the compound of formula (II)) is shown below:

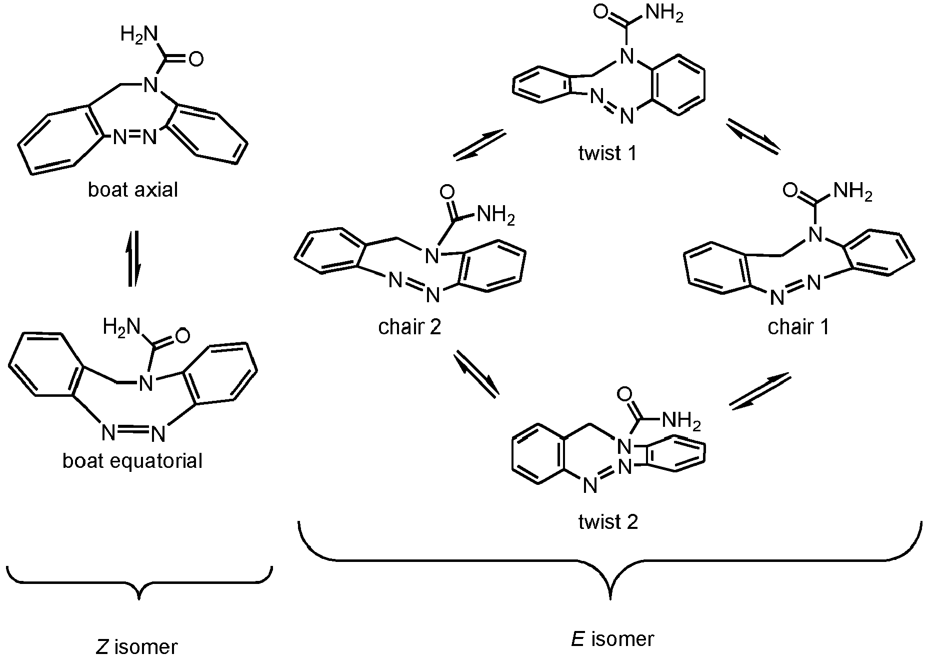

In a preferred embodiment, in the compound of formula (I), G¹ is CR¹, G² is CR², G³ is CR³, G⁴ is CR⁴, G⁵ is CR⁵ and G⁶ is CR⁶. Preferably, wherein one of R¹, R², R³, R⁴, R⁵, R⁶, R⁷ and R⁸ is a halogen atom and the rest are hydrogen atoms. More preferably wherein one of R¹, R², R³, R⁴, R⁵, R⁶, R⁷ and R⁸ is a fluorine atom and the rest are hydrogen atoms. Even more preferably wherein all of R¹, R², R³, R⁴, R⁵, R⁶, R⁷ and R⁸ are hydrogen atoms.

In another preferred embodiment, in the compound of formula (I), no more than one of G¹, G², G³, G⁴, G⁵ and G⁶ is a nitrogen atom.

In a particular embodiment, in the compound of formula (I), one of G¹, G², G³, G⁴, G⁵ and G⁶ is a nitrogen atom.

In another particular embodiment, in the compound of formula (I), G¹ is a nitrogen atom, G² is CR², G³ is CR³, G⁴ is CR⁴, G⁵ is CR⁵ and G⁶ is CR⁶. Preferably, wherein one of R², R³, R⁴, R⁵, R⁶, R⁷ and R⁸ is a halogen atom and the rest are hydrogen atoms. More preferably wherein one of R², R³, R⁴, R⁵, R⁶, R⁷ and R⁸ is a fluorine atom and the rest are hydrogen atoms. Even more preferably wherein all of R², R³, R⁴, R⁵, R⁶, R⁷ and R⁸ are hydrogen atoms.

In another particular embodiment, in the compound of formula (I), G¹ is CR¹, G² is a nitrogen atom, G³ is CR³, G⁴ is CR⁴, G⁵ is CR⁵ and G⁶ is CR⁶. . Preferably, wherein one of R¹, R³, R⁴, R⁵, R⁶, R⁷ and R⁸ is a halogen atom and the rest are hydrogen atoms. More preferably wherein one of R¹, R³, R⁴, R⁵, R⁶, R⁷ and R⁸ is a fluorine atom and the rest are hydrogen atoms. Even more preferably wherein all of R¹, R³, R⁴, R⁵, R⁶, R⁷ and R⁸ are hydrogen atoms.

In another particular embodiment, in the compound of formula (I), G¹ is CR¹, G² is CR², G³ is a nitrogen atom, G⁴ is CR⁴, G⁵ is CR⁵ and G⁶ is CR⁶. Preferably, wherein one of R¹, R², R⁴, R⁵, R⁶, R⁷ and R⁸ is a halogen atom and the rest are hydrogen atoms. More preferably wherein one of R¹, R², R⁴, R⁵, R⁶, R⁷ and R⁸ is a fluorine atom and the rest are hydrogen atoms. Even more preferably wherein all of R¹, R², R⁴, R⁵, R⁶, R⁷ and R⁸ are hydrogen atoms.

In another particular embodiment, in the compound of formula (I), G¹ is CR¹, G² is CR², G³ is CR³, G⁴ is a nitrogen atom, G⁵ is CR⁵ and G⁶ is CR⁶. Preferably, wherein one of R¹, R², R³, R⁵, R⁶, R⁷ and R⁸ is a halogen atom and the rest are hydrogen atoms. More preferably wherein one of R¹, R², R³, R⁵, R⁶, R⁷ and R⁸ is a fluorine atom and the rest are hydrogen atoms. Even more preferably wherein all of R¹, R², R³, R⁵, R⁶, R⁷ and R⁸ are hydrogen atoms.

In another particular embodiment, in the compound of formula (I), G¹ is CR¹, G² is CR², G³ is CR³, G⁴ is CR⁴, G⁵ is a nitrogen atom and G⁶ is CR⁶. Preferably, wherein one of R¹, R², R³, R⁴, R⁶, R⁷ and R⁸ is a halogen atom and the rest are hydrogen atoms. More preferably wherein one of R¹, R², R³, R⁴, R⁶, R⁷ and R⁸ is a fluorine atom and the rest are hydrogen atoms. Even more preferably wherein all of R¹, R², R³, R⁴, R⁶, R⁷ and R⁸ are hydrogen atoms.

In another particular embodiment, in the compound of formula (I), G¹ is CR¹, G² is CR², G³ is CR³, G⁴ is CR⁴, G⁵ is CR⁵ and G⁶ is a nitrogen atom. Preferably, wherein one of R¹, R², R³, R⁴, R⁵, R⁷ and R⁸ is a halogen atom and the rest are hydrogen atoms. More preferably wherein one of R¹, R², R³, R⁴, R⁵, R⁷ and R⁸ is a fluorine atom and the rest are hydrogen atoms. Even more preferably wherein all of R¹, R², R³, R⁴, R⁵, R⁷ and R⁸ are hydrogen atoms.

In another preferred embodiment, in the compound of formula (I), G¹ is a nitrogen atom, G² is CR², G³ is CR³, G⁴ is CR⁴, G⁵ is selected from the group consisting of nitrogen atom and CR⁵, and G⁶ is selected from the group consisting of nitrogen atom and CR⁶, with the proviso that no more than one of G⁵ and G⁶ are nitrogen atoms. Preferably, wherein one of R², R³, R⁴, R⁵ (when present, i.e. when G⁵ is CR⁵), R⁶ (when present, i.e. when G⁶ is CR⁶), R⁷ and R⁸ is a halogen atom and the rest are hydrogen atoms. More preferably wherein one of R², R³, R⁴, R⁵ (when present), R⁶ (when present), R⁷ and R⁸ is a fluorine atom and the rest are hydrogen atoms. Even more preferably wherein all of R², R³, R⁴, R⁵ (when present), R⁶ (when present), R⁷ and R⁸ are hydrogen atoms.

In particular embodiment, in the compound of formula (I), G¹ is a nitrogen atom, G² is CR², G³ is CR³, G⁴ is CR⁴, G⁵ is a nitrogen atom, and G⁶ is CR⁶. Preferably, wherein one of R², R³, R⁴, R⁶, R⁷ and R⁸ is a halogen atom and the rest are hydrogen atoms. More preferably wherein one of R², R³, R⁴, R⁶, R⁷ and R⁸ is a fluorine atom and the rest are hydrogen atoms. Even more preferably wherein all of R², R³, R⁴, R⁶, R⁷ and R⁸ are hydrogen atoms.

In another particular embodiment, in the compound of formula (I), G¹ is a nitrogen atom, G² is CR², G³ is CR³, G⁴ is CR⁴, G⁵ is s CR⁵, and G⁶ is a nitrogen atom. Preferably, wherein one of R², R³, R⁴, R⁵, R⁷ and R⁸ is a halogen atom and the rest are hydrogen atoms. More preferably wherein one of R², R³, R⁴, R⁵, R⁷ and R⁸ is a fluorine atom and the rest are hydrogen atoms. Even more preferably wherein all of R², R³, R⁴, R⁵, R⁷ and R⁸ are hydrogen atoms.

In another preferred embodiment, in the compound of formula (I), G¹ is CR¹, G² is CR², G³ is a nitrogen atom, G⁴ is CR⁴, G⁵ is CR⁵ and G⁶ is CR⁶. Preferably, wherein one of R¹, R², R⁴, R⁵, R⁷ and R⁸ is a halogen atom and the rest are hydrogen atoms. More preferably wherein one of R¹, R², R⁴, R⁵, R⁷ and R⁸ is a fluorine atom and the rest are hydrogen atoms. Even more preferably wherein all of R¹, R², R⁴, R⁵, R⁷ and R⁸ are hydrogen atoms.

The halogen atoms that may be present in groups R¹, R², R³, R⁴, R⁵, R⁶, R⁷ and R⁸ of the compound of formula (I) of the present invention may be any halogen atom as previously defined, preferably a fluorine atom.

In a preferred embodiment, in the compound of formula (I), zero, one or two of R¹, R², R³, R⁴, R⁵, R⁶, R⁷ and R⁸ are halogen atoms and the rest are hydrogen atoms. More preferably, zero, one or two of R¹, R², R³, R⁴, R⁵, R⁶, R⁷ and R⁸ are fluorine atoms and the rest are hydrogen atoms.

In a preferred embodiment, in the compound of formula (I), zero or one of R¹, R², R³, R⁴, R⁵, R⁶, R⁷ and R⁸ are halogen atoms and the rest are hydrogen atoms. More preferably, zero or one of R¹, R², R³, R⁴, R⁵, R⁶, R⁷ and R⁸ are fluorine atoms and the rest are hydrogen atoms.

In a particular embodiment, in the compound of formula (I), one of R¹, R², R³, R⁴, R⁵, R⁶, R⁷ and R⁸ is a halogen atom and the rest are hydrogen atoms. More preferably, one of R¹, R², R³, R⁴, R⁵, R⁶, R⁷ and R⁸ is a fluorine atom and the rest are hydrogen atoms.

In a particular embodiment, in the compound of formula (I), all of R¹, R², R³, R⁴, R⁵, R⁶, R⁷ and R⁸ are hydrogen atoms.

In a preferred embodiment, in the compound of formula (I), G¹ is selected from nitrogen atom and CH, G² is CH, G³ is selected from nitrogen atom and CH, G⁴ is CH, G⁵ is selected from nitrogen atom and CH, G⁶ is CR⁶ wherein R⁶ is selected from hydrogen and halogen atom, R⁷ is hydrogen and R⁸ is selected from hydrogen and halogen atom.

In a preferred embodiment, the compound of formula (I) is a compound of formula (II): 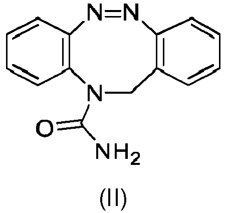 or pharmaceutically acceptable salt or stereoisomer thereof.

### Synthesis of compounds of formula (I)

Compounds of formula (I) may be synthesized by using some of the methods described below and summarized in Scheme 1 below, as well as other processes known in the field of the organic chemistry.

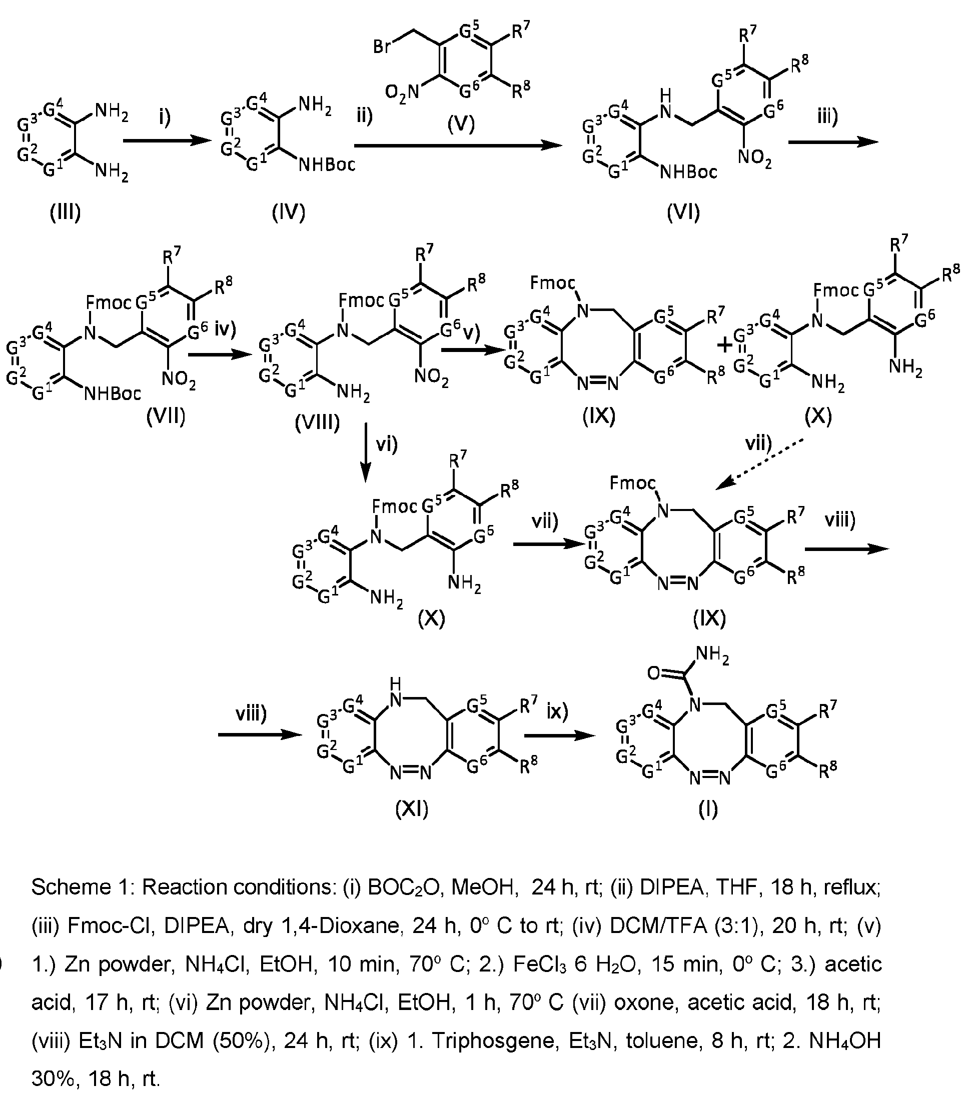

The first step in the process is the Boc (tert-butoxycarbonyl) protection of one of the primary amine groups in a compound of formula (III) to give the Boc-derivative (IV), wherein G¹, G², G³ and G⁴ are as defined for the compounds of formula (I) [Lentes et al., J. Am. Chem. Soc., 2019, 141(34), 13592-13600]. This reaction can be carried out following common procedures in the art for Boc protection, such as by reaction with Boc anhydride (di-tert-butyl dicarbonate, Boc₂O), in particular using methanol as the solvent. A substitution reaction may be carried out on the Boc-derivative of formula (IV) with a bromo-derivative of formula (V) to give a compound of formula (VI) in the presence of a base such as diisopropyl ethyl amine (DIPEA) in an organic solvent, such as tetrahydrofuran (THF) [Lentes et al., J. Am. Chem. Soc., 2019, 141(34), 13592-13600], wherein G¹, G², G³, G⁴, G⁵, G⁶, R⁷ and R⁸ are as defined for the compounds of formula (I). The NH group in compound (VI) may be protected with a Fmoc (fluorenylmethyloxycarbonyl) group to give a compound of formula (VII), for example by reaction with 9-fluorenylmethoxycarbonyl chloride (Fmoc-CI) in the presence of a base such as DIPEA and in an organic solvent such as 1,4-dioxane [Lentes et al., J. Am. Chem. Soc., 2019, 141(34), 13592-13600], wherein G¹, G², G³, G⁴, G⁵, G⁶, R⁷ and R⁸ are as defined for the compounds of formula (I). Next, the Boc-protected amine group in the compound of formula (VII) is deprotected using common procedures in the art for Boc-deprotection, such as treatment with an acid (in particular trifluoroacetic acid (TFA) in the presence of an organic solvent (in particular dichloromethane (DCM)), yielding a compound of formula (VIII) [Lentes et al., J. Am. Chem. Soc., 2019, 141(34), 13592-13600], wherein G¹, G², G³, G⁴, G⁵, G⁶, R⁷ and R⁸ are as defined for the compounds of formula (I). Cyclization of the compound of formula (VIII) may be carried out by two different procedures. The first one is a one-pot synthesis using the Baeyer-Mills reaction wherein the nitro group in the compound of formula (VIII) is reduced with zinc/NH₄Cl to form the corresponding hydroxylamine followed by oxidation with iron (III) chloride to the nitroso derivative; after, glacial acetic acid may be added to give the Fmoc-protected diazocine intermediate of formula (IX) [Lentes et al., J. Am. Chem. Soc., 2019, 141(34), 13592-13600], wherein G¹, G², G³, G⁴, G⁵, G⁶, R⁷ and R⁸ are as defined for the compounds of formula (I). As a side product of this one-pot synthesis, the Fmoc-protected diamine compound (X) is obtained, wherein G¹, G², G³, G⁴, G⁵, G⁶, R⁷ and R⁸ are as defined for the compounds of formula (I). This side product (X) can be recovered and cyclized to give the Fmoc-protected diazocine intermediate of formula (IX) using the reaction conditions described below for the second procedure. The second procedure, involves firstly the reduction of the nitro group in the compound of formula (VIII) to an amine group to give the compound of formula (X) wherein G¹, G², G³, G⁴, G⁵, G⁶, R⁷ and R⁸ are as defined for the compounds of formula (I). This reduction may be carried out by treatment with zinc/NH₄Cl. Next, the cyclization of compound (X) to give the Fmoc-protected diazocine intermediate of formula (IX) is performed by treatment with oxone in the presence of acetic acid, wherein G¹, G², G³, G⁴, G⁵, G⁶, R⁷ and R⁸ are as defined for the compounds of formula (I). The next step is removal of the Fmoc-protecting group in the Fmoc-protected diazocine intermediate of formula (IX) obtained by either the first or the second procedure to give diazocine intermediate of formula (XI), wherein G¹, G², G³, G⁴, G⁵, G⁶, R⁷ and R⁸ are as defined for the compounds of formula (I). Fmoc deprotection can be carried out using common procedures in the art, such as by treatment with a base such as trimethylamine (EtsN) in an organic solvent such as DCM [Lentes et al., J. Am. Chem. Soc., 2019, 141(34), 13592-13600]. The final carboxamination of the diazocine intermediate of formula (XI) may be carried out with a multistep reaction by adding triphosgene in basic conditions followed by hydrolyzation with NH₄OH affording the compounds of formula (I), wherein G¹, G², G³, G⁴, G⁵, G⁶, R⁷ and R⁸ are as previously defined.

### Pharmaceutical compositions comprising the compound of formula (I)

In a second aspect, the invention is directed to a pharmaceutical composition comprising a compound of formula (I) as defined above, and a pharmaceutically acceptable excipient.

The term "pharmaceutically acceptable excipient" refers to a vehicle, diluent, or adjuvant that is administered with the active ingredient. Such pharmaceutical excipients can be sterile liquids, such as water and oils, including those of petroleum, animal, vegetable, or synthetic origin, such as peanut oil, soybean oil, mineral oil, sesame oil, and similar, surfactant, bile acid, chelating agents, preservatives, cyclodextrins, among others. Water or saline aqueous solutions and aqueous dextrose and glycerol solutions, particularly for injectable solutions, are preferably used as vehicles. Suitable pharmaceutical vehicles are described in "Remington's Pharmaceutical Sciences" by E.W. Martin, 21st Edition, 2005. Other suitable excipients are cyclodextrins and polyethylene glycols (e.g. liquid PEGs such as PEG₄₀₀). Surfactants, bile acids, chelating agents, preservatives, and cyclodextrins increase chemical stability and bioavailability and decrease local irritation [Brian G. Short, Toxicologic Pathology, 2008, 36(1), 49-62].

Compounds formula (I) according to the present invention may be administered by the topical, oral, sublingual, parenteral, subcutaneous, intramuscular, intravenous, transdermal, intranasal, and/or rectal routes.

The compounds may be administered alone or in combination with one or more other compounds of the invention or one or more other drugs. In general, the compounds should be administered as a formulation in association with one or more pharmaceutically acceptable excipients. Any administration method commonly used for drugs, such as solutions (including eye drops), cream, ointment, powder, tablets, coated tablets, capsules, syrup, powder, and suppository, may be used. The pharmaceutical composition can be formulated employing conventional liquid or solid vehicles or diluents and pharmaceutical additives, according to the desired mode of administration. The mentioned formulations will be prepared using standard methods, such as those described or referred to in the Spanish, E.U., and U.S. Pharmacopoeias and similar reference texts.

The compounds of formula (I) may be administered in a therapeutically effective amount. "Effective" amount or a "therapeutically effective amount" of a compound is meant a nontoxic but sufficient amount of the drug or agent to provide the desired effect. The amount that is "effective" will vary from subject to subject, depending on the age and general condition of the individual, the particular active agent or agents, and the like. Thus, it is not always possible to specify an exact "effective amount". However, an appropriate "effective" amount in any individual case may be determined by one of ordinary skill in the art using routine experimentation. The compounds will typically be administered once or more times a day, for example 1, 2, 3 or 4 times daily.

### Medical uses of the compound of formula (I)

Neurological conditions such as epilepsy and neuropathic pain involve excessive neuronal activity or signaling. As shown in the examples, the present compounds of formula (I) are capable of inhibiting or decreasing the frequency of action potentials generation and are therefore suitable for the treatment of neurological conditions that involve excessive neuronal activity or signaling, such as epilepsy and neuropathic pain. Moreover, the compounds of formula (I) are light activated and, thus, the neuroinhibitory effects are only localized at the region of illumination and on demand, decreasing the incidence of the severe adverse effects. As shown in the examples, in zebrafish larvae, the compounds of the invention are not toxic, do not alter zebrafish larvae behaviour in the dark, and produce behavioural responses upon illumination.

Therefore, in a third aspect, the present invention provides a compound of formula (I) as defined above, or a pharmaceutical composition comprising a compound of formula (I) as defined above, for use as a medicament.

The invention also relates to the use of a compound of formula (I) as defined above, or a pharmaceutical composition comprising a compound of formula (I) as defined above, for the manufacture of a medicament.

In a fourth aspect, the present invention provides a compound of formula (I) as defined above, or a pharmaceutical composition comprising a compound of formula (I) as defined above, for use in the treatment and/or prevention of epilepsy (in particular epileptic focal seizures) and neuropathic pain (in particular peripheral nerve injury, painful polyneuropathy, postherpetic neuralgia, painful radiculopathy, trigeminal neuralgia, brachial plexus injury, phantom limb pain).

The present invention also relates to the use of a compound of formula (I) as defined above, or a pharmaceutical composition comprising a compound of formula (I) as defined above, for manufacturing a medicament for the treatment and/or prevention of epilepsy (in particular epileptic focal seizures) and neuropathic pain (in particular peripheral nerve injury, painful polyneuropathy, postherpetic neuralgia, painful radiculopathy, trigeminal neuralgia, brachial plexus injury, phantom limb pain).

The invention also relates to a method of treatment and/or prevention of a subject in need thereof of epilepsy (in particular epileptic focal seizures) and neuropathic pain (in particular peripheral nerve injury, painful polyneuropathy, postherpetic neuralgia, painful radiculopathy, trigeminal neuralgia, brachial plexus injury, phantom limb pain), which comprises the administration of a compound of formula (I) as defined above, or a pharmaceutical composition comprising a compound of formula (I) as defined above.

The terms "treat", "treatment", or "treatment of" as used herein refer to reducing the potential for a certain disease or disorder, reducing the occurrence of a certain disease or disorder, and/or a reduction in the severity of a certain disease or disorder, preferably, to an extent that the subject no longer suffers discomfort and/or altered function due to it. It also refers to mitigating or decreasing at least one clinical symptom and/or inhibition or delay in the progression of the condition and/or prevention or delay of the onset of a disease or illness.

The terms "prevention", "preventing" or "prevent" as used herein refer to avoiding the appearance of a certain disease or disorder. The prevention can be complete (e.g. the total absence of a disease). The prevention can also be partial, such that for example the occurrence of a disease in a subject is less than that which would have occurred without the administration of the combination or composition of the present invention. Prevention also refers to reduced susceptibility to a clinical condition. The prevention also includes reducing the risk of suffering the disease.

The subject to which the compounds and pharmaceutical compositions described herein are administered is a human or animal; preferably subjects are mammals, more preferably humans.

The term "epilepsy" as used herein refers to a central nervous system (neurological) disorder in which brain activity becomes abnormal, causing seizures or periods of unusual behaviour, sensations and sometimes loss of awareness. When seizures appear to result from abnormal activity in just one area of the brain, they are called focal seizures. These seizures fall into two categories: (i) focal seizures without loss of consciousness (these seizures do not cause a loss of consciousness; they may alter emotions or change the way things look, smell, feel, taste or sound; some people experience dejà vu; this type of seizure may also result in involuntary jerking of one body part, such as an arm or leg, and spontaneous sensory symptoms such as tingling, dizziness and flashing lights), and (ii) focal seizures with impaired awareness (these seizures involve a change or loss of consciousness or awareness; this type of seizure may seem like being in a dream; during a focal seizure with impaired awareness, the affected subject may stare into space and not respond normally to the environment or perform repetitive movements, such as hand rubbing, chewing, swallowing or walking in circles). Seizures that appear to involve all areas of the brain are called generalized seizures. Six types of generalized seizures exist: (i) absence seizures (they are characterized by staring into space with or without subtle body movements such as eye blinking or lip smacking and only last between 5-10 seconds; these seizures may occur in clusters, happening as often as 100 times per day, and cause a brief loss of awareness), (ii) tonic seizures (they cause stiff muscles and may affect consciousness; these seizures usually affect muscles in the back, arms and legs and may cause the affected subject to fall to the ground), (iii) atonic seizures (they cause a loss of muscle control; since this most often affects the legs, it often causes the affected subject to suddenly collapse or fall down), (iv) clonic seizures (they are associated with repeated or rhythmic, jerking muscle movements; these seizures usually affect the neck, face and arms), (v) myoclonic seizures (they usually appear as sudden brief jerks or twitches and usually affect the upper body, arms and legs), and (vi) tonic-clinic seizures (they can cause an abrupt loss of consciousness and body stiffening, twitching and shaking; they sometimes cause loss of bladder control or biting of the tongue).

The expression "neuropathic pain" as used herein refers to pain caused by damage or injury to the nerves that transfer information between the brain and spinal cord from the skin, muscles and other parts of the body. The pain is usually described as a burning sensation and affected areas are often sensitive to the touch. Symptoms of neuropathic pain may also include excruciating pain, pins and needles, difficulty correctly sensing temperatures and numbness. Examples of neuropathic pain are peripheral nerve injury, painful polyneuropathy, postherpetic neuralgia, painful radiculopathy, trigeminal neuralgia, brachial plexus injury, and phantom limb pain.

As explained above and as shown in the examples, the compounds of formula (I) are inactive or exhibit a low inhibitory activity of action potentials in the dark, whereas they convert into an active isomer upon illumination. Thus, for carrying out the medical uses and methods described above, the compound of formula (I) is administered to the subject (preferably systemically, such as by oral route). Then, illumination is applied at the seizure focal spot (for epilepsy) or at the spinal cord (for pain) by means of one of these methods:
(1) direct illumination with an external light source (e.g. LED patch or wearable device) with wavelength that penetrates tissue (orange or red, e.g., between 580 nm and 750 nm);
*(2) in situ* illumination with an implanted light source that is remotely actuated by electromagnetic waves (e.g. wireless micro LED device available commercially, such as the optogenetics implants NeuroLux described in https://www.tse-systems.com/service/neurolux-optogenetics/) if the emitted wavelength has a short penetration in tissue (violet, blue, or green, i.e. between 350 nm and 550 nm); or
(3) direct illumination with multiphoton excitation using external pulsed infrared laser and suitable optic devices (such as described by Izquierdo-Serra, M. et al., J. Am. Chem. Soc., 2014, 136(24), 8693-8701; and Riefolo, F. et al., J. Am. Chem. Soc., 2019, 141(18), 7628-7636).

In all cases, the therapeutic effects of the compounds of formula (I) are released locally and on demand (upon seizure onset or pain sensation) without concomitant adverse effects in non-illuminated regions of the body.

The following examples represent specific embodiments of the present invention. They do not intend to limit in any way the scope of the invention defined in the present description.

### EXAMPLES

### 1. Synthesis and photochemical characterization

### 1.1. Abbreviations

DCM: dichloromethane, MeOH: methanol; EtOAc: ethyl acetate; EtOH: ethanol; DMSO: dimethylsulfoxide; THF: tetrahydrofuran; MeCN: acetonitrile.

### 1.2. Materials and methods

### • Reagents:

All reagents and solvents were purchased from Sigma-Aldrich and Cymit Quimica S.L. and were used without any further purification. Reactions were monitored by analytical thin layer chromatography (TLC) on commercial silica gel 60 F254 aluminum foils 20 x 20 cm (Merck). Spots were visualized under 254 or 365 nm UV lamp and/or with the use of an appropriate stain (ninidrine solution, Dragendorff reagent, alkaline potassium permanganate solution or phosphomolybdic acid solution 5% in ethanol). Compounds were purified by flash chromatography on PanReac Applichem silica gel 60 (40-63 µm) as stationary phase; the mobile phases are specified for each compound. All the UV/vis spectra and experiments were registered with Shimadzu UV-1800 UV-VIS spectrophotometer using quartz cuvettes (10 mm light path).

### • NRM:

Nuclear Magnetic Resonance (NMR) spectra were recorded with a Varian Mercury 400 MHz instrument and tetramethylsilane (TMS) and/or residual signals of deuterated solvents were used as internal standard (CDCl3: 1H 7.26 ppm, 13C 77.16 ppm; CD3OD 1H 3.31 ppm, 13C 49.00 ppm, DMSO-d6 1H 2.50 ppm, 13C 39.52 ppm). Chemical shifts (δ) are expressed as parts-per-million (ppm) and coupling constants (J) are reported in hertz (Hz).

### • HPLC and mass spectrometry:

High-performance Liquid Chromatography (HPLC) analysis were performed with a Waters Alliance e2695 Separation Module paired with a Waters 2998 UV/vis Photodiode Array Detector (Diode array 190-800 nm) and a Waters AQUITY QDa Mass Detector (positive ionization ESI+). Samples were injected on a XSelect CSH C18 3.5 mm 4.6x50 mm analytical column with the following mobile phases, respectively called line A and B: water with formic acid 0.1% (v/v) and acetonitrile with formic acid 0.1% (v/v). The injections were eluted with the following method: flow 1.6 mL/min, gradient 0.00-4.50 min, 5-100% B; 4.50-5.00 min, 100-5% B.

The purity of all the final compounds has been tested by high-performance mass spectroscopy (HPLC) analysis integrating the peaks at the Diode Array scanning from 190 to 800 nm.

### • Photochemical characterization:

The photochromic response was characterized by the absorption spectra after irradiation with LEDs at λ = 365 nm, 380 nm, 420 nm, 460 nm, 500 nm. The maximal changes in the spectra were associated to the optimal λ to isomerize from Z to E and vice versa. Resistance to photofatigue was tested by cycles of illuminations between the optimal λ. The kinetics of the spontaneous relaxation in the dark (time course of absorption at a fixed λ) was measured to quantify the relaxation lifetime by fitting the plots to an exponential decay curve. The UV-vis absorption analysis for the photochromic characterization has been carried out at room temperature. Stock solutions were prepared in the concentration of 10 mM in DMSO. The solutions used for the measurements were obtained by diluting the stock solutions in PBS at the desired final concentration (in the range of 30 µM and 50 µM). Finally, the photostationary states (PSS) were quantified using HPLC at the λ corresponding to the isosbestic point.

### • HPLC switching experiments:

The quantification of photostationary states (PSS) has been measured by HPLC analysis. The peaks at the Diode Array have been detected and integrated at the wavelength corresponding to the isosbestic point.

### 1.3. Synthesis of tert-butyl (2-aminophenyl)carbamate

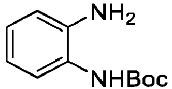

Tert-butyl (2-aminophenyl)carbamate was synthesized following the procedure reported by Lentes et al. [J. Am. Chem. Soc., 2019, 141(34), 13592-13600] with a 67% yield.

### 1.4. Synthesis of tert-butyl (2-((2-nitrobenzyl)amino)pheny/)carbamate

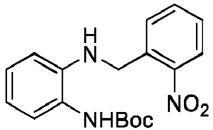

Tert-butyl (2-((2-nitrobenzyl)amino)phenyl)carbamate was synthesized following the procedure reported by Lentes et al. [J. Am. Chem. Soc., 2019, 141(34), 13592-13600] with a 85% yield.

### 1.5. Synthesis of (9H-fluoren-9-yl)methyl(2-((tert-butoxycarbonyl)amino)phenyl)(2-nitrobenzyl)carbamate

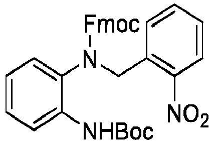

DIPEA (0.5 mL, 2.92 mmol) and Fmoc-CI (1.888 g, 7.3 mmol) were added under nitrogen to an iced cooled solution of *tert*-butyl (2-((2-nitrobenzyl)amino)phenyl)carbamate (1 g, 2.92 mmol) in 80 mL of 1,4-dioxane anhydrous. The mixture of reaction was stirred at room temperature for 3 days with daily additions of 1 equivalent of Fmoc-CI (755 mg, 2.92 mmol). The reaction was quenched by addition of 50 mL of saturated NaCl solution and 50 mL of ethyl acetate. The aqueous phase was extracted with EtOAc (3 x 30 mL) then the organic layers were combined, dried over MgSO₄ and concentrated under vacuum. The crude was purified by flash chromatography on silica (ethyl acetate/cyclohexane 0 - 30 %) to obtain the product as a yellow solid (1.56 g, 94%). The characterization of the compound was as reported by Lentes et al. [J. Am. Chem. Soc., 2019, 141(34), 13592-13600].

### 1.6. Synthesis of (9H-fluoren-9-yl)methyl (2-aminophenyl)(2-nitrobenzyl)carbamate

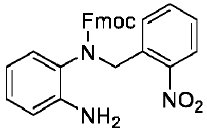

(9H-Fluoren-9-yl)methyl (2-aminophenyl)(2-nitrobenzyl)carbamate was synthesized following the procedure reported by Lentes et al. [J. Am. Chem. Soc., 2019, 141(34), 13592-13600] with a 68 % yield.

### 1.7. Synthesis of (9H-fluoren-9-yl)methyl (2-aminobenzyl)(2-aminophenyl)carbamate

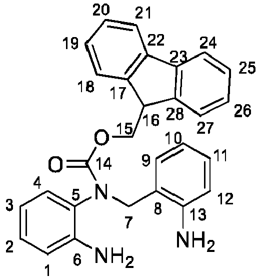

To a solution of (9*H*-fluoren-9-yl)methyl (2-aminophenyl)(2-nitrobenzyl)carbamate (500 mg, 1.07 mmol) in 27 mL of ethanol at 70°C was added 5 mL of NH₄Cl 2M and zinc powder (259 mg, 3.96 mmol). The reaction mixture was stirred at 70°C for 1 h. The solvent was evaporated, the residue was suspended in 50 mL of NaHCOs saturated aq. solution and extracted with DCM (3 x 50 mL). The organic layers were combined, dried over MgSO₄ and concentrated under vacuum. The crude was purified by flash chromatography on silica (ethyl acetate/cyclohexane 0 - 40 %) in order to afford the product as an orange solid (317.6 mg, 68%). MS (ES⁺) *m*/*z* 436.19 [M+H]⁺. ¹H NMR: (500 MHz, CDCl₃) δ 7.70 (d, *J* = 7.6 Hz, 2H, Ar-H-21-24), 7.35 (t, *J* = 7.3 Hz, 2H, Ar-H-18-27), 7.23 - 7.01 (m, 6H, Ar-H-11-4-25-26-19-20), 6.96 (d, *J* = 7.8 Hz, 1H, Ar-H-3), 6.78 (t, *J* = 7.5 Hz, 1H, Ar-H-2), 6.74 - 6.62 (m, 3H, Ar-H-9-12-1), 6.55 (t, *J* = 7.4 Hz, 1H, Ar-H-10), 5.02 (d, *J* = 14.7 Hz, 1H, C-H-7), 4.59 (d, *J* = 14.7 Hz, 1H, C-H-7), 4.44 - 4.24 (m, 2H, C-H-15), 4.07 (t, *J* = 7.9 Hz, 1H, C-H-16), 3.70 (s, 2H, N*H*). ¹³C NMR: (101 MHz, cdcl₃) δ 157.10 (Ar-C-14), 146.15 (Ar-C), 144.77 (Ar-C), 144.25 (Ar-C-17), 143.99 (Ar-C-28), 141.66 (Ar-C-23), 141.60 (Ar-C-22), 132.40 (Ar-C-1), 129.94 (Ar-C-4), 129.81 (ArC-3), 129.55 (Ar-C), 129.26 (Ar-C), 128.01 (Ar-C), 127.98 (Ar-C), 127.34 (Ar-C), 127.28 (Ar-C), 126.58 (Ar-C), 125.94 (Ar-C), 125.73 (Ar-C), 120.73 (Ar-C-8), 120.20 (Ar-C-21-24), 118.91 (Ar-C-2), 118.09 (Ar-C-10), 116.72 (Ar-C-12), 115.89 (Ar-C-9), 68.65 (-C-15), 51.37 (-C-7), 47.41 (-C-16).

### 1.8. Synthesis of (9H-fluoren-9-yl)methyl (Z)-dibenzo[c,g][1,2,5]triazocine-11(12H)-carboxylate

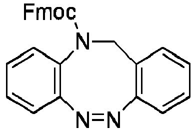

### • Procedure A, as reported by Lentes et al. [J. Am. Chem. Soc. 2019, 141(34), 13592-13600]:

To a solution of (9H-fluoren-9-yl)methyl (2-aminophenyl)(2-nitrobenzyl)carbamate (605 mg, 1.30 mmol) in 33 mL of ethanol at 70 °C were added 6 mL of NH₄Cl 2 M and zinc-powder (314 mg, 4.8 mmol). The mixture was stirred at 70 °C for 10 min and then hot filtrated. The collected solution was warmed to 40 °C and added dropwise to an ice cooled solution of iron(III) chloride hexahydrate (703 mg, 2.6 mmol) in 4 mL of water and 20 g of ice. The solution was stirred at 0 °C for 15 min, then 52 mL of acetic acid was added. The reaction was stirred overnight at room temperature and afterwards it was neutralized with NaHCOs. The water phase was extracted with DCM (3 x 70 mL) and the combined organic layers were dried over MgSO₄ and the solvent was concentrated. The crude was purified by flash chromatography on silica (ethyl acetate/cyclohexane 0 - 30 %) in order to afford the product as a yellow solid (71.6 mg, 13%) and the side product (9H-fluoren-9-yl)methyl (2-aminophenyl)(2-nitrobenzyl)carbamate as an orange solid (359.2 mg).

### • Procedure B:

Oxone (848 mg, 2.76 mmol) was added in portions to a solution of (9H-fluoren-9-yl)methyl (2-aminophenyl)(2-aminobenzyl)carbamate (600 mg, 1.38 mmol) in 300 mL of acetic acid. The reaction mixture was stirred for 4 days with daily additions of 1 equivalent of oxone (310 mg, 1.38 mmol). The solvent was evaporated and the residue was dissolved in DCM and washed with NaHCOs saturated solution. The organic layer was dried over MgSO₄ and concentrated under vacuum. The crude was purified by flash chromatography on silica (ethyl acetate/cyclohexane 0 - 25 %) to afford the product as an orange solid (350.4 mg, 59%). MS (ES⁺) *m*/*z* 432.07 [M+H]⁺. NMR data as reported by Lentes et al. [J. Am. Chem. Soc. 2019, 141(34), 13592-13600].

### 1.9. Synthesis of (Z)-11,12-dihydrodibenzo[c,g][1,2,5]triazocine

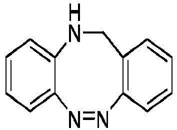

*(Z)-11,12-dihydrodibenzo[c,g][1,2,5]triazocine* was synthesized following the procedure reported by Lentes et al. [J. Am. Chem. Soc., 2019, 141(34), 13592-13600] with a 89% yield.

### 1.10. Synthesis of (Z)-dibenzo[c,g][1,2,5]ltriazocine-11(12H)-carboxamide (compound of formula (II), LC-098)

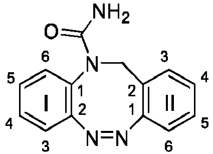

To solution of (Z)-11,12-dihydrodibenzo[c,g][1,2,5]triazocine 10 mg, 0.05 mmol) in toluene (2 mL) at 10°C were added TEA (0.004 mL, 0.017 mmol) and triphosgene (5 mg, 0.017 mmol). The mixture of reaction was stirred at 10°C and monitored by TLC until the starting material was fully consumed. Afterwards, were added 2 mL of aq. NH₃ 30% and the mixture was stirred overnight. The reaction mixture was concentrated at rotavapor and the crude was purified by flash chromatography on silica (dichloromethane/methanol 0 - 5%). MS (ES⁺) *m*/*z* 253.29 [M+H]⁺. ¹H NMR (400 MHz, CDCl₃) δ 7.24 (m, 2H, Ar^{I}-*H-*3-6), 7.16 (dd, *J* = 4.0, 0.9 Hz, 2H, Ar^{II}-*H*-*3*-6), 7.08 (dd, *J* = 4.0, 0.9 Hz, 2H, Ar^{II}-*H*-*4*-5), 6.92 (dt, *J* = 7.6, 1.0 Hz, 1H, Ar^{I}-*H*-4), 6.89 (d, *J* = 7.8 Hz, 1H, Ar^{I}-*H*-5), 5.03 - 4.37 (m, 4H, -N*H*₂, -C*H*₂). ¹³C NMR (101 MHz, CDCl₃) δ 153.63, 130.06, 129.14, 129.11, 128.87, 128.65, 127.77, 124.09, 119.84, 119.16, 52.12. Purity of final compound (Z)-*dibenzo[c,g][1,2,5]triazocine-11(12H)-carboxamide (compound of formula (II), LC-098)* is ≥ 95%.

### 1.11. Results

The compound of formula (II) was subjected to photochemical characterization using the methods previously described. As shown in Figure 1, the photoconversion from an isomer to the other is reversible and can be repeated for several cycles of illumination without degradation or loss in photochromic properties. Thermal back isomerization has been recorded leaving the compounds to relax in the dark over time.

In the dark, at benchtop conditions, the compound is fully converted into the Z inactive isomer as shown in Figure 4A and 4B.

The absorption maxima of the dark-adapted *Z* isomer can be excited at 400 nm (blue light) to achieve a mixture of 49% *Z* and 51% *E* as shown in Figures 5A and 5B. In this case, the large separation between the *Z* and *E* nπ* absorption bands of the compound of formula (II) allowed to selectively excite the *E* isomer with irradiation at ≥ 500 nm obtaining the full back-isomerization to the *Z* inactive form (Figure 6A and 6B). Quantitatively recovering the dark-adapted configuration is a desirable feature as it allows to have full control over the activation/inactivation of the drug. Notably, in the compound of formula (II) the nπ* band of the *E* isomer is tailoring in the near infrared thus the *E* to *Z* isomerization can be achieved by using light in the range of 500-590 nm. The use of red and infra-red light in photopharmacology is highly desirable for its reduced toxicity compared to UV light and the increased permeation in biological tissues. Hence, we irradiated the *E* isomer using a 590 nm LED leading to quantitative conversion to the Z isomer over 1 h irradiation. The same experiment has been repeated with a Volpi Intralux 4000 light source, equipped with a 90 W EPV halogen lamp with a broad emission band between 600-700 nm and a colour temperature of 3150 K that allows to effectively excite the *E* nπ* diazocine band. The full conversion is achieved after one minute irradiation. The back-isomerization is reversible with green light or by spontaneous thermal relaxation in dark conditions. The half-life for relaxation in the dark is t1/2 = 3.2 h. The configurational changes are reversible and repeatable for several cycles of illumination without loss in absorption or degradation.

### 2. Biological activity

### 2.1. Culturing of primary hippocampal neuros

Procedures were performed in accordance with the European guidelines for animal care and use in research and were approved by the Animal Experimentation Ethics Committee at the University of Barcelona. Sprague-Dawley rat pups (P 1-5) were sacrificed by decapitation, hippocampi were isolated and treated with 0.1% trypsin in HBSS (10 min, 37°C). Neurons were plated on Poly-D-Lysine (PDL)-coated 16 mm coverslips (0.5-1 x 10⁵ cells) and incubated at 37°C, 5% CO₂ for 1,5 hours (to allow cells adhesion) in MEM supplemented with heat-inactivated FBS (5%), heat-inactivated HS (5%), penstrep (10 Ul/ml), L-glutamine (2mM) and glucose (20mM). Cells were cultured in Neurobasal A medium, supplemented with B-27 (5%), penstrep (5 Ul/ml), glutaMAX (0,5x) and glucose (15mM). On DIV 3 cultured cells were treated with 1 µM of Ara-C to prevent proliferation of non-neuronal cells. 50% of the maintenance medium was exchanged every 4 days. Electrophysiology experiments were performed at neurons older than 17 DIV which demonstrated intrinsic firing activities.

### 2.2. Electrophysiologycalrecordings

Whole-cell recordings were performed at room temperature using an EPC-10 amplifier (HEKA Elektronik, Germany) and Patch Master software (HEKA). Cells were bathed with external solution containing: 150 mM NaCl, 3 mM KCI, 2 mM MgCl₂, 2 mM CaCl₂, 10 mM HEPES, 10 mM D-glucose; pH was adjusted to 7.40-7.42 with NaOH. Recording pipettes were pulled from borosilicate glass capillaries (Harvard Apparatus Ltd, USA) and had resistance 5-10 MOhms. Solution used for filling patch pipettes to record neuronal firing contained (mM): 115 mM K-gluconate, 10 mM KCI, 10 mM HEPES, 10 mM EGTA, 5 mM Mg-ATP, 0.5 mM Na-GTP, pH adjusted to 7.3 with KOH (290-295 mOsmol). Firing of primary hippocampal neurons was recorded in current clamp configuration.

### 2.3. Drug application

Drugs were first dissolved in DMSO and then in the bath solution till the final concentration.

### 2.4. Data analysis

Data was analyzed using Igor Pro 6.05 (WaveMatrics, USA) and Origin 8.0 (OriginLab, USA) software. Results are represented as mean ± SD. Statistical difference between groups was evaluated with Mann-Whitney test and was considered significant at the value of P below 0.05.

### 2.5. Zebrafish

### • Animal housing:

Wild-type zebrafish embryos (Tupfel long-fin strain) were purchased from the animal facility of the Barcelona Biomedical Research Park (PRBB) and raised in darkness for 7 days at 28 °C in UV filtered tap water in Petri dishes (daily cleaned and refilled). Animal development was checked every 24 hours. Unhealthy or abnormal embryos and larvae were removed and euthanatized in tricaine methanosulfonate (400 mg/l). All experiments and procedures were conducted according to the European Directive 2010/63/EU.

### • Assay protocol:

Behavioral studies were conducted on wild-type zebrafish larvae at 7 days post-fertilization (dpf). Vehicle and compounds were added with a multichannel pipette to exclude differences related to a delay in the application of each solution. Movements were recorded and analyzed using the ZebraBox tracking system and the ZebraLab software (ViewPoint Life Science, France). On the morning of the test, 7-dpf larvae were moved into a new batch of fresh water and checked for motility capabilities and possible physical mutations. Larvae were then randomly divided into control and treatment groups. Each individual was placed in a separate well of a 96-well plate, each containing 200 µl of fresh UV filtered water and left undisturbed in the dark for 30 min to get acquainted with the new setting (habituation time). Afterwards, 100 µL of water were removed from each well and replaced with 100 µl of a double concentrated vehicle or treatment solution. At this point, the plate was inserted into the ZebraBox and the recording period started. Animals were exposed to controlled cycles of dark, 380 nm, 420 nm, 500 nm wavelengths or with a halogen lamp (warm white light), using the illumination protocols represented by coloured-shaded areas in their respective graphs (see figures). Illumination at 380, 420 and 500 nm was performed with a built-in array of 12 LEDs placed 12 cm away from the multiwell plate. The halogen lamp used was the Volpi *Intralux 4000* (115 W). Light intensities, measured with a Newport 1916-C optical power meter coupled to a Newport 918D-SL-OD3R detector, were 0.21 mW·cm⁻² (380 nm), 2.43 mW·cm⁻² (420 nm) and 0.69 mW·cm⁻² (500 nm). All experiments were conducted between 12.00 and 15.00 pm (UTC+01:00).

### • Tracking and analysis of swimming activity:

Alterations of locomotor activity were determined by monitoring and measuring fast movements, swimming distances and duration of high-speed swimming. In particular, the dependent variables measured included: total distance travelled, distance travelled at ≤ 2 mm·s⁻¹, distance travelled at 2-6 mm·s⁻¹, distance travelled at ≥ 6 mm·s⁻¹, time spent moving, time spent freezing, and number of bursts. The arbitrary cut-off for motility (6 mm·s⁻¹) was chosen because no variance was found among the groups at lower speeds. Figures show distances travelled over 6 mm·s⁻¹ and total movements (addition of movements below cut-off high speed swimming) were not considered to limit the error of the video recording on minimal movements. Data were analysed by two-way ANOVA with Tukey's post-hoc test for statistical significance (GraphPad Prism 6).

### 2.6. Results

The compound of formula (II) adapts a Z conformation in darkness and was expected to exert its inhibitory action upon switching to the E-state by 400 nm light. Figure 2A demonstrates that generation of action potentials by hippocampal neurons can be efficiently and repeatedly suppressed by blue light illumination during application of the compound of formula (II). When applied to spontaneously firing neurons, 30 µM of the compound of formula (II) decreased the frequency of the action potentials generation to 72±5% from control (P > 0.05; Fig 2B), while under blue light (400 nm) illumination, firing decreased to 28±13% from control (P < 0.01; n=4).

*In vitro* active photoswitchable compound of formula (II) was evaluated in the early stage of zebrafish (*Danio rerio*) larvae. The compound was diluted in zebrafish water and applied to 24 developed free-swimming larvae (7 days postfertilisation) and analysed under varying light conditions. The compound of formula (II) produced a dose-dependent effect on swimming behaviour which could be controlled using two visible light wavelengths. Zebrafish larvae show preference towards visible wavelengths, such as 420 and 500 nm [Oliveira, J. et al., A. (2015). Zebrafish, 12(2), 166-173.; Kysil, E. V. et al., Zebrafish, 2017, 14, 197-208], with periodic side-to-side tail movements and darting behaviours. Light ON and OFF activities are also described in literature and increase in swimming activity is expected when light is turned OFF after prolonged illumination periods [Emran, F., Rihel, J. & Dowling, J. E. A behavioral assay to measure responsiveness of Zebrafish to changes in light intensities. J. Vis. Exp. (2008) doi: 10.3791/923], as observed in Figure 3A (55^{th} to 60^{th}, 65^{th} to 70^{th}, 75^{th} to 80^{th} and 85^{th} to 90^{th} minutes, see black control trace). In two different groups, treatment with the compound of formula (II) induced no effects during the first 20 minutes of the experiment, suggesting no trans-pharmacological effect. Once illuminated with 420 nm, thus increasing Z isomerisation of the compound of formula (II), a cumulative dose-dependent effect appeared. Lowest dosage (1 µM) induced a significant increase of the distance swum in larval zebrafish after the third illumination with blue light (420 nm), maintaining it for the rest of this light periods. Contrarily, the highest dose (50 µM) produced the opposite cumulative effect, where during the first blue light illumination periods the larvae were significantly hyperactive, but the effect was reduced with consecutive blue illumination. The mid 10 µM concentration produced a constant effect for both lights, increasing the activity of the larvae under blue light and returning to control levels under green illumination (500 nm). All concentrations did not alter significantly the swimming distances of the larva when illuminated with green light (when comparing each period individually to the control group; only 10 µM was slightly increased when the average of all green periods were considered). Illumination with a warm white light (halogen lamp, patterned shaded areas Figure 3A) also blocked the hyperactivity of all the treated larvae immediately. No signs of toxicity or distress were observed in the larvae.

## Claims

1. A compound of formula (I): 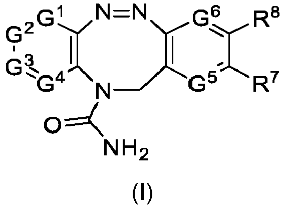 wherein:
G¹ is selected from nitrogen atom and CR¹,
G² is selected from nitrogen atom and CR²,
G³ is selected from nitrogen atom and CR³,
G⁴ is selected from nitrogen atom and CR⁴,
G⁵ is selected from nitrogen atom and CR⁵,
G⁶ is selected from nitrogen atom and CR⁶,
R¹, R², R³, R⁴, R⁵, R⁶, R⁷ and R⁸ are independently selected from hydrogen and halogen atom,
with the proviso that no more than one of G¹, G², G³ and G⁴ is a nitrogen atom, and
with the proviso that no more than one of G⁵ and G⁶ is a nitrogen atom,
or a pharmaceutically acceptable salt or stereoisomer thereof.

2. A compound according to claim 1 wherein G¹ is CR¹, G² is CR², G³ is CR³, G⁴ is CR⁴, G⁵ is CR⁵ and G⁶ is CR⁶.

3. A compound according to claim 1 wherein no more than one of G¹, G², G³, G⁴, G⁵ and G⁶ is a nitrogen atom.

4. A compound according to claim 1 wherein G¹ is a nitrogen atom, G² is CR², G³ is CR³, G⁴ is CR⁴, G⁵ is selected from the group consisting of nitrogen atom and CR⁵, and G⁶ is selected from the group consisting of nitrogen atom and CR⁶, with the proviso that no more than one of G⁵ and G⁶ are nitrogen atoms.

5. A compound according to claim 3 wherein G¹ is CR¹, G² is CR², G³ is a nitrogen atom, G⁴ is CR⁴, G⁵ is CR⁵ and G⁶ is CR⁶.

6. A compound according to any one of claims 1 to 5 wherein the halogen atom is a fluorine atom.

7. A compound according to any one of claims 1 to 6 wherein zero, one or two of R¹, R², R³, R⁴, R⁵, R⁶, R⁷ and R⁸ are halogen atoms and the rest are hydrogen atoms.

8. A compound according to claim 7 wherein zero or one of R¹, R², R³, R⁴, R⁵, R⁶, R⁷ and R⁸ is a halogen atom and the rest are hydrogen atoms.

9. A compound according to claim 8 wherein one of R⁶ and R⁸ is a halogen atom and R¹, R², R³, R⁴, R⁵ and R⁷ are hydrogen atoms.

10. A compound according to claim 1 wherein G¹ is selected from nitrogen atom and CH, G² is CH, G³ is selected from nitrogen atom and CH, G⁴ is CH, G⁵ is selected from nitrogen atom and CH, G⁶ is CR⁶ wherein R⁶ is selected from hydrogen and halogen atom, R⁷ is hydrogen and R⁸ is selected from hydrogen and halogen atom.

11. A compound according to any one of claims 1, 2, 7 and 8 having formula (II): 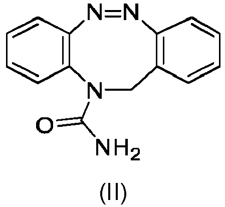 and pharmaceutically acceptable salts thereof.

12. Pharmaceutical composition comprising a compound according to any one of claims 1 to 11, and a pharmaceutically acceptable excipient.

13. Compound according to any one of claims 1 to 11 or pharmaceutical composition according to claim 12, for use in medicine.

14. Compound according to any one of claims 1 to 11 or pharmaceutical composition according to claim 12, for use in the treatment and/or prevention of a disease selected from the group consisting of epilepsy and neuropathic pain.

15. Compound or pharmaceutical composition for use according to claim 14, wherein the neuropathic pain is selected from the group consisting of peripheral nerve injury, painful polyneuropathy, postherpetic neuralgia, painful radiculopathy, trigeminal neuralgia, brachial plexus injury, and phantom limb pain; and/or wherein epilepsy is an epileptic focal seizure.
